# EUROPÄISCHE PATENTANMELDUNG

(11) **EP 1 589 338 A2**
(43) Veröffentlichungstag der Anmeldung: **26.10.2005**
(21) Anmeldenummer: 05013646.4
(22) Anmeldetag: 23.09.2002
(51) Int. Cl.: G01N 33/18, G01N 17/00

(54) **Verfahren zur Verminderung von Schäden an Heizungsanlagen und flüssiger, schwefelarmer Brennstoff**

(30) Priorität: 21.09.2001 EP 01810927
(62) Teilanmeldung aus: 02762199.4
(71) Anmelder: Swiss E-Technic AG, 7303 Mastrils (CH)
(72) Erfinder: Füllemann, Jörg, 7303 Mastrils (CH)
(74) Vertreter: Walder, Martin Bernhard

(57) **Zusammenfassung**

Die Erfindung betrifft ein Verfahren zur Vermeidung von Erosionsschäden in Heizungsanlagen infolge einer Verbrennung von schwefelarmem Brennstoff, mit einem Massenanteil an Schwefel von höchstens 0,05%, wobei auf den Schaden verursachenden Prozess Einfluss genommen wird, indem eine während der Verbrennung des Brennstoffs im Verbrennungsraum vorhandene elektrische Spannung gering gehalten oder vermindert wird oder indem die Bildung von Carbonsäure während der Verbrennung des Brennstoffs im Verbrennungsraum vermindert wird. Die Erfindung betrifft ferner einen flüssigen schwefelarmen Brennstoff, mit einem Massenanteil an Schwefel von höchstens 0,05% mit einem zugesetzten Additiv zum Verhindern oder Abbauen von elektrostatischen Ladungen.

## Beschreibung

In jüngster Zeit sind schwefelarme Heizöle auf den Markt gekommen, die einen Massenanteil an Schwefel von 0,05 bis 0,001 % aufweisen. Bei der Verwendung solcher schwefelarmer Heizöle treten bisher unbekannte Schadensbilder an den Metallteilen im Kesselraum und am Ölbrenner, insbesondere am Flammbecher auf. Die Schadensbilder treten auch in Brennwert-Heizanlagen auf, die die Kondensationswärme nutzen und mit Niedertemperatur-Brennern, sog. Gebläse-Blaubrennern ausgerüstet sind. Die Schadensbilder zeigen scharf umrissene, kraterförmig erodierte Stellen mit einer fein gekörnten Oberfläche und unterscheiden sich deutlich von einer Hochtemperaturkorrosion. Derartige Schadensbilder können bereits innerhalb weniger Wochen nach Inbetriebnahme der Heizungsanlage entstehen. Die Wahl von korrosionsbeständigeren Materialien hat nur bedingt Einfluss auf die Schadensbilder.

Es ist daher Aufgabe der Erfindung Mittel und Wege zu finden, die neuartigen Schadensbilder an Heizungsanlagen bei der Verwendung von Heizöl mit einem Schwefelgehalte von 0,05 und weniger zu verhindern oder zu vermindern.

In einer Versuchsreihe mit sieben Brennstoffmarken unterschiedlicher Herkunft konnte festgestellt werden, dass die Herkunft des Brennstoffs entscheidenden Einfluss hat auf seine erosive Wirkung. Heizöle mit identischem Schwefelgehalt, jedoch unterschiedlicher Herkunft zeigten unterschiedliche Erosivität. Vergleiche der chemischen und physikalischen Analysen der unterschiedlichen Brennstoffe ergaben bisher keine schlüssigen Resultate über die Ursache der unterschiedlichen Erosivität. Es wird vermutet, dass der Raffinerieprozess oder die Ölquelle die Heizölqualität derart beeinflusst, dass eine Erosion auftritt oder nicht auftritt.

Erfindungsgemäss wird daher vorgeschlagen, zur Vermeidung der Erosionsschäden in Heizungsanlagen den flüssigen, schwefelarmen Brennstoff, z.B. chargenweise durch eine neutrale Stelle oder in der Raffinerie, auf seine potentielle erosive Wirkung beim Verbrennen in Heizungsanlagen zu prüfen. Zur Prüfung des Brennstoffes wird vorteilhaft der Brennstoff in Verbrennungsluft nahe einer Prüffläche vernebelt und der Brennstoffnebel einem elektrischen Spannungsfeld ausgesetzt und entflammt.

Weshalb dabei an metallischen Prüfflächen eine von blossem Auge sichtbare Erosion bereits nach kurzer Zeit auftritt, ist im Detail nicht bekannt. Es kann dies jedoch folgendermassen erklärt werden: Bekanntlich entsteht bei der Verbrennung von schwefelarmem Heizöl in der Dampf/Flüssigphase und bei Anwesenheit von Sauerstoff Carbonsäure. Es wird vermutet, dass diese Carbonsäure die Metalloxidschicht (überwiegend Eisenoxid), die das Metall schützt, angreift und zerstört und an diesen Stellen ohne Metalloxidschicht das blanke Metall erodiert wird. Es wird weiter angenommen, dass bei der Zündung solch erosiven Heizöls in Gegenwart des elektrischen Spannungsfeldes des Zündfunkens Ionen des Plasmas, gleich anschliessend an deren Entstehung, gegen die Metallfläche schiessen und bei ihrem Aufprall diese erodieren.

Es konnte festgestellt werden, dass bei einer mit einer Kohlenschicht versehenen Prüffläche Schadensbilder rascher auftreten als bei blanken Prüfflächen. Scheinbar wird die Bildung von Carbonsäure durch die Anwesenheit einer Kohlenschicht auf der metallischen Oberfläche begünstigt. Die Kohlenschicht braucht dabei lediglich einige µ stark zu sein. Eine ausreichende Schichtstärke wird auf 10 bis 15 µ geschätzt.

Es konnte ferner festgestellt werden, dass das Durchfahren eines Temperaturbereiches notwendig ist. Dieser Temperaturbereich liegt sicher zwischen 350 und 600 °C. Ein eingeschränkter Temperaturbereich liegt zwischen 370 und 550 °C. In diesem Temperaturbereich treten die Prozesse auf, die das Schadensbild verursachen. Aufgrund von weiteren Untersuchungen ist dieser Temperaturbereich möglicherweise noch präziser einschränkbar. Es konnte weiter beobachtet werden, dass die Schadensbilder beschleunigt an Stellen auftreten, die durch den Brennstoff benetzt werden.

Ein Prüfungsverfahren zur Prüfung von flüssigem Brennstoff auf dessen potentielle erosive Wirkung beim Verbrennen in Heizungsanlagen zeichnet sich vorteilhaft durch eine mehrfache Wiederholung einer Sequenz von Verfahrensschritten aus, welche Sequenz folgende Merkmale aufweist:
- Vernebeln von Brennstoff und vermischen von Brennstoffnebel und Verbrennungsluft,
- ein elektrisches Spannungsfeld errichten und den Brennstoffnebel dem Spannungsfeld aussetzen.
- Zünden des versprühten Brennstoffes, so dass ein Plasma entsteht,
- Aufrechterhalten des elektrischen Spannungsfelds und das Plasma dem elektrischen Spannungsfeld aussetzen,
- Abstellen der Brennstoffzufuhr.

Dabei wird vorteilhaft beim Starten des Prüfverfahrens Heizöl verwendet, das eine zum Starten geeignete Temperatur aufweist. Sobald der Verbrennungsprozess Hitze entwickelt, wird vorteilhaft auf gekühltes Heizöl umgestellt. Das gekühlte Heizöl kann vorteilhaft mit einem Fliessverbesserer versetzt sein, damit es möglichst kühl, z.B. bei minus 10 °C verwendet werden kann. Dank einem gekühlten Brennstoff wird die Prüffläche möglichst lange benetzt.

Obige Sequenz kann vorteilhaft mit einem weiteren Verfahrensschritt ergänzt werden, in welchem vorhandene Prüfflächen gekühlt werden. Zum Kühlen nach Abstellen der Brennstoffzufuhr wird vorteilhaft Luft verwendet.

Zur Überprüfung des Ionenbeschusses wird vorteilhaft eine metallene Prüffläche verwendet. Diese ist zweckmässigerweise aus einem für den Brennerbau geeigneten Material, z. B. Alloy 601, gefertigt. Die Prüffläche ist vorteilhaft in einen Brenner, z.B. in einen Verdampfer eingesetzt. Die Prüffläche kann die innere Oberfläche eines Flammbechers sein. Sie kann auch die Oberfläche eines Verdampfers sein. Sie kann insbesondere auch eine Oberfläche eines herkömmlichen Brenners sein.

Die Zündung erfolgt vorteilhaft durch einen Zündfunken. Dadurch können für die Zündung die Elektroden verwendet werden, die zur Errichtung eines Spannungsfelds ohnehin benötigt werden, und das Spannungsfeld ist in diesem Fall mit Sicherheit schon vorhanden, wenn das Plasma entsteht. Die Elektroden können mit einer Wechselspannung versorgt sein, oder eine konstante Potentialdifferenz aufweisen. Das Spannungsfeld kann zwischen zwei Elektroden aufgebaut werden, während die Prüffläche geerdet ist. Es kann auch zwischen einer Elektrode und der Prüffläche aufgebaut werden, so dass die Prüffläche eine Elektrode bildet. Diese die Prüffläche bildende Elektrode ist vorteilhaft aus Flachmaterial gefertigt.

In einer Heizanlage erfolgt eine Ölzufuhr vorschriftsmässig erst nach einer Vorzündzeit von 10 bis 15 Sekunden. Dies kann beim erfindungsgemässen Prüfverfahren ebenso gehandhabt werden. Wesentlich ist, dass das Plasma einem elektrischen Spannungsfeld ausgesetzt sind. Vorzugsweise ist das Spannungsfeld während dem Entstehen der Flamme bereits aufgebaut.

Es wurde gefunden, dass je länger in einer Heizanlage die Nachzündzeit eingestellt ist und der Zündfunken bzw. das Spannungsfeld gleichzeitig mit der Flamme aufrechterhalten bleibt, desto grösser ist die erodierende Wirkung eines Schadenbilder hervorrufenden Brennstoffes an benachbarten Metallteilen. Vorteilhaft wird daher beim Prüfverfahren in einer Sequenz während mehr als 35 sec., vorteilhaft bis zum Abstellen der Ölzufuhr, ein elektrisches Spannungsfeld aufrechterhalten.

Da die Erosion nahe der Elektrode oder Elektroden grösser ist als an entfernteren Stellen, sind beim Prüfverfahren die Elektroden oder ist die Elektrode vorteilhaft in einem Abstand von weniger als 50 mm, vorteilhaft in einem Abstand von weniger als 15 mm, oder gar in einem Abstand von 3 bis 7 mm von der metallenen Prüffläche, bzw. der Stelle der Prüffläche, nahe der die Ionisation des Brennstoffes geschieht. Die Spannung und der Abstand zwischen den Spannungspotentialen sind einander reziprok entsprechend derart zu wählen, dass ein möglichst grosses Volumen des Plasmas einer möglichst hohen Spannung ausgesetzt ist.

Je höher die Spannung des Spannungsfeldes ist, desto höher ist die Wahrscheinlichkeit, dass der Schadenbilder hervorrufende Brennstoff ein solches Schadenbild hervorruft. Daher wird das Plasma vorteilhaft einer Spannung von 2 mal 7500 V und mehr, vorzugsweise von wenigstens 2 mal 10'000 V, besonders bevorzugt von wenigstens 2 mal 15'000 Volt ausgesetzt. Es konnte jedoch festgestellt werden, dass einzelne Brennstoffe schon bei Spannungen von 200 V oder 230 V Schadenbilder hervorrufen.

Es konnte an Heizungsanlagen für schwefelarmes Heizöl extra leicht (Schwefelgehalt < 0.5 ppm) beobachtet werden, dass im Sprühschatten der Zündelektroden weniger Schadensbilder auftreten. Daher wird beim Prüfverfahren vorteilhaft eine im Flammenbereich angeordnete Metallfläche mit dem Heizöl besprüht oder gar benetzt. Damit die Ionisation des Brennstoffes möglichst lange nahe der Prüffläche eintritt, wird der Flammbecher oder eine andere Prüffläche vor dem Beginn einer neuen Sequenz vorteilhaft auf eine Temperatur unter 250°C, vorzugsweise unter 220°C, besonders bevorzugt unter 200°C abgekühlt. Eine Kühlung geschieht vorzugsweise durch Nachlüften während einigen Sekunden oder Minuten. Die Prüffläche ist zwecks Kühlung vorteilhaft axial im Frischluftstrom angeordnet. Vorteilhaft wird die Prüffläche während der gesamten Brenndauer gekühlt. Durch die Abkühlung zwischen den Sequenzen unter 250 Grad wird erreicht, dass bei der nächsten Sequenz die Prüffläche einen Temperaturbereich von 350 bis 550 oder 600 °C durchlaufen muss. Durch die Wahl der Sequenzlänge wird erreicht, dass die Prüffläche 550 bis 600 Grad erreicht, jedoch der Prozess nicht unnötig lange bei heisseren und daher weniger effizienten Temperaturen weitergeführt wird.

Je kürzer die Verbrennungsphase einer Sequenz dauert, desto weniger erhitzt sich die Umgebung der Flamme. Je länger das Prüfverfahren bei niedriger Temperatur des Flammbechers oder einer anderen Prüffläche abläuft, desto grösser ist der Zeitanteil, während dem die erodierende Metallfläche benetzt wird und die Bedingungen für die erodierende Wirkung des Brennstoffes optimal sind. Daher wird vorteilhaft eine Sequenz kurz gewählt, so dass sie weniger als 15 Minuten dauert, vorteilhaft weniger als 10 Minuten, besonders bevorzugt weniger als 5 Minuten. Dadurch kann der erosionswirksame Zeitanteil pro Stunde erhöht werden.

Mit einem solchen Verfahren kann eine Heizöl-Probe innerhalb von höchstens 30 bis 40 Betriebsstunden (Brenndauer) auf ihre erodierende Wirkung überprüft werden. Es ist zu erwarten, dass durch eine Optimierung des Prüfverfahrens diese Betriebszeit noch weiter verkürzt werden kann. Je nach Eigenschaften der Probe tritt eine erste von blossem Auge sichtbare Erosion bereits nach wenigen Betriebsstunden auf.

Eine erodierte oder nach z.B. 40 Stunden noch immer nicht erodierte Prüffläche wird zweckmässigerweise als Beleg der Heizölqualität einer Charge archiviert. Für jede zu prüfende Charge wird daher vorteilhaft eine neue Prüffläche in einer Prüfvorrichtung angeordnet. Diese Prüffläche kann vorgängig mit einer Kohlenschicht versehen werden. Es kann aber auch beim Starten des Prozesses die Verbrennung derart beeinflusst werden, dass sich eine Kohlenschicht auf der Prüffläche bildet.

Zur Prüfung von Heizöl oder anderen flüssigen Brennstoffen auf deren potentielle erosive Wirkung beim Verbrennen in Heizungsanlagen, insbesondere in Brennwert-Heizungsanlagen, d.h. bei Heizanlagen, in denen bei der Verbrennung entstehender Wasserdampf an der Kesselwandung kondensiert, wird erfindungsgemäss eine Vorrichtung verwendet, welche wenigstens folgendes aufweist: Einen Brennstoffbehälter für eine Brennstoffprobe, eine an den Brennstoffbehälter angeschlossenen Brennstoffpumpe, eine nach der Brennstoffpumpe angeordnete Düse zum Versprühen von Brennstoff, einen Spannungswandler, wenigstens eine mit dem Spannungswandler verbundene Elektrode, einen Ventilator für eine Frischluftzufuhr, eine Steuerung zur Steuerung der Brennstoffpumpe, des Spannungswandlers und des Ventilators, sowie eine einfach aus der Vorrichtung entfernbare und in die Vorrichtung einsetzbare Prüffläche. Bei einer solchen Vorrichtung kann ein Feuerungsraum oder Kessel gänzlich fehlen. Vorteilhaft ist jedoch ein Temperaturfühler zum Messen der Temperatur der Prüffläche vorhanden. Vorteilhaft ist ferner die Prüffläche metallisch und bevorzugt mit einem Kohlenstofffilm versehen.

Es wurde gefunden, dass eine Erosion der Prüffläche an Stellen, die Spuren von Filzstiften aufwiesen oder an Prüfflächen, die mit Werkzeugen aus Buntmetall bearbeitet wurden rascher auftrat. Es wird daraus geschlossen, dass Metalloxide, insbesondere Oxide von Nicht-Eisen-Metallen, an der Oberfläche der Prüffläche die Erosion begünstigen. Daher wird die Prüffläche vor dem Prüfverfahren vorteilhaft mit einem Metalloxid behandelt. Die Prüffläche kann auch mit Metallstaub behandelt werden, der dann bei der Verbrennung von Brennstoff oxidiert.

Die Steuerung ist vorteilhaft derart ausgelegt, dass sie eine Vielzahl der beschriebenen Sequenzen automatisch steuert.

Der Spannungswandler ist dabei vorteilhaft derart dimensioniert, dass er eine Spannung von zweimal mehr als 7'500 V, vorzugsweise zweimal mehr als 10'000 V, besonders bevorzugt von zweimal mehr als 15'000 Volt generiert. Herkömmliche Spannungswandler für Heizungsanlagen sind auf eine Spannung von zweimal 7500 Volt, eine Vorzündzeit von 12 sec. und eine Nachzündzeit von ca. 20 sec. ausgelegt. Für eine Prüfvorrichtung ist er jedoch vorteilhaft derart ausgelegt, dass er die Spannung über einen Zeitraum von mehr als 35 sec., vorzugsweise mehr als einer Minute, besonders bevorzugt praktisch unbegrenzt aufrecht erhalten kann. Die Spannung kann auch gegenüber der Erde als zweitem Potential aufgebaut werden. Es kann auch, allenfalls zusätzlich zur Spannung zwischen den Elektroden, eine konstante Potenzialdifferenz zwischen einer separaten Elektrode und der geerdeten oder unter Spannung gesetzten Prüffläche aufrecht erhalten werden. Es kann auch die Prüffläche gegenüber der Erde unter Spannung gesetzt werden. Durch eine dadurch erreichte Spannungsdifferenz zwischen der Prüffläche und anderen Teilen in der Umgebung des Plasmas kann der Ionenfluss gesteuert werden.

Die Elektrode ist, bzw. die Elektroden sind vorteilhaft in einem Abstand von weniger als 50 mm, vorteilhaft in einem Abstand von weniger als 15 mm von einer metallenen Prüffläche angeordnet. Vorteilhaft ist ein Temperaturfühler vorhanden, mit dem die Temperatur der Prüffläche überwacht werden kann.

Die Düse und die Prüffläche sind vorteilhaft so angeordnet und die Düse weist eine derartige Charakteristik auf, dass gewährleistet ist, dass der Brennstoff gegen die im Bereich der Flamme angeordnete Prüffläche spritzt und der Brennstoff die Prüffläche benetzt und nahe der Prüffläche in ein Plasma übergeht. Es kann eine erste Düse für eine die Flamme aufrechterhaltende Brennstoffzufuhr zur Flamme und eine zweite Düse zur Besprühung oder Benetzung der Prüffläche vorhanden sein. Die metallene Prüffläche ist vorteilhaft aus einem Edelstahl gefertigt, z.B. Alloy 601, DIN 2.4851, der gewährleistet, dass keine Hochtemperaturkorrosion auftritt. Dadurch ist ein auftretendes Schadensbild eindeutig der Erosion und der Qualität des geprüften Brennstoffs zuzuordnen.

Zur Vermeidung des Schadens kann nicht nur die Erosivität des Brennstoffs überprüft werden. Es können dem Brennstoff bei Bedarf, d.h. bei zu hoher Erosivität einer Charge, Antioxidantien beigemischt werden. Antioxidatien verhindern, wie auch ein Schwefelgehalt von über 0,4 bis 0,5%m/m, die Bildung von Carbonsäure. Ferner kann dem Heizöl ein Additiv zugesetzt werden, das gewährleistet, dass die elektrostatischen Ladungen bei der Verbrennung gering bleiben. Ein solches "Static Dissipator Additive" ist beispielsweise das bisher in Flugbezin verwendete "Stadis 450". Ob die Dosierung der Additive ausreicht wird vorteilhaft mittels des beschriebenen Prüfverfahrens überprüft.

Es wird auf Grund der Feststellung der erhöhten Erosivität des Brennstoffes bei Anwesenheit von Metalloxiden auch vermutet, dass zur Entschwefelung des Brennstoffes eingesetzte Metalloxide in Spuren im Brennstoff verbleiben und eine Erosion begünstigen. Daher kann zur Verhinderung der Erosion der Metalloxidgehalt der im Brennstoff reduziert oder deren Aggressivität durch eine Zugabe von Antioxidantien neutralisiert werden. Es wird daher angenommen, dass die Bemessung der Zugabemenge von Antioxidantien aufgrund einer Messung des Metalloxidgehalts des Brennstoffes vorgenommen werden kann.

Eine weitere Massnahme zur Verhinderung der beschriebenen Erosion besteht darin, den Brennstoff mit einer Glühzündung zu entzünden und so die elektrische Spannung im Brennraum möglichst gering zu halten. Ein vorteilhafter Blaubrenner ist daher mit einer Glühzündung, insbesondere mit einer Niedervolt-Glühzündung ausgerüstet. Dadurch wird verhindert, dass Ionen in grosser Zahl in einem Spannungsfeld beschleunigt werden, auf das Metall aufprallen und dadurch dessen Oberfläche erodieren.

Alle diese Massnahmen basieren auf der Erkenntnis, dass auf den die zu vermeidenden Schadensbilder verursachenden Prozess in zweierlei Hinsicht Einfluss genommen werden kann, nämlich: 1. durch Verminderung einer elektrischen Spannung im Verbrennungsraum, und 2. durch die Verminderung der Bildung von Carbonsäure. Fehlt lediglich einer der genannten Faktoren, so ist das Schadensrisiko bereits wesentlich vermindert. Wie und weshalb die Brennstoffeigenschaften, die in jeder Charge wieder anders sein können, bei dem schadenden Prozess beteiligt sind, ist noch nicht erkennbar.

### Kurzbeschreibung der Figuren:

Es zeigt:
- Fig. 1: eine teilweise geschnittene Seitenansicht eines Brenners für eine erfindungsgemässe Prüfvorrichtung,
- Fig. 2: Seitenansicht des Brennerkopfes,
- Fig. 3: Frontalansicht des Brennerkopfes,
- Fig. 4: eine geschnittenen Darstellung des Brennerkopfes,
- Fig. 5: eine schematische Darstellung einer Prüfvorrichtung.

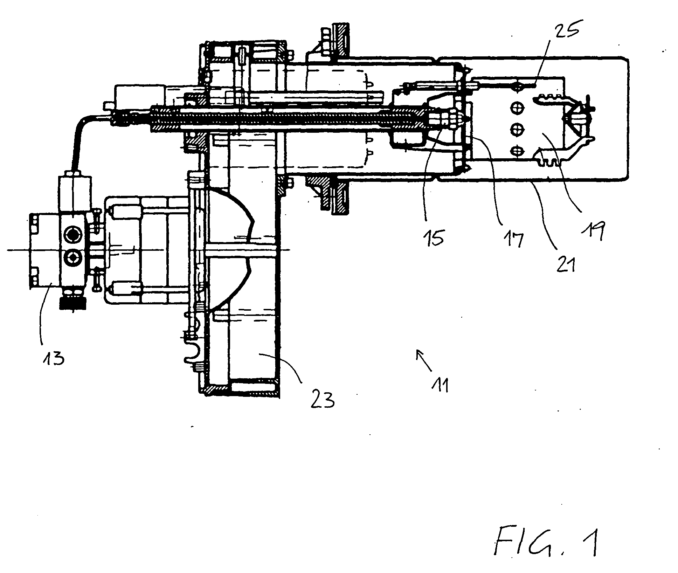

Der Brenner 11 gemäss Figur 1 weist eine Brennstoffpumpe 13 auf, mit der der Brennstoff aus einem nicht dargestellten Behälter der Brennstoffdüse 15 zugepumpt wird. Die Brennstoffdüse 15 ist mit ihrer Düsenöffnung etwa in der Ebene einer Stauscheibe 17 angeordnet. In der Stauscheibe 17 ist eine zentrale Öffnung vorhanden, durch die der Brennstoff in einen Verdampfer 19 gespritzt werden kann. Der Verdampfer 19 ist in einem Flammrohr 21 angeordnet.

Der Brenner 11 weist zudem einen Ventilator 23 auf. Mit dem Ventilator 23 kann Frischluft mit einem bestimmten Druck an die Stauscheibe 17 herangeführt werden. Die Frischluft tritt in der Folge in den Innenraum des Verdampfers 19 ein, wo sie sich mit verdampftem Brennstoff und mit rezirkulierten Verbrennungsgasen vermischt. Nicht dargestellt sind Temperaturfühler zur Überwachung der Temperatur des Brennstoffes und der Prüffläche. Nicht dargestellt ist ein Verbrennungsraum, in welchem die Flamme brennt.

Erkennbar sind zwei Zündelektroden 25 nahe der Vergaserwandung. Diese sind an einen nicht dargestellten Spannungswandler angeschlossen, der die Elektroden mit einer alternierenden Potentialdifferenz von zweimal 7500 Volt versorgt.

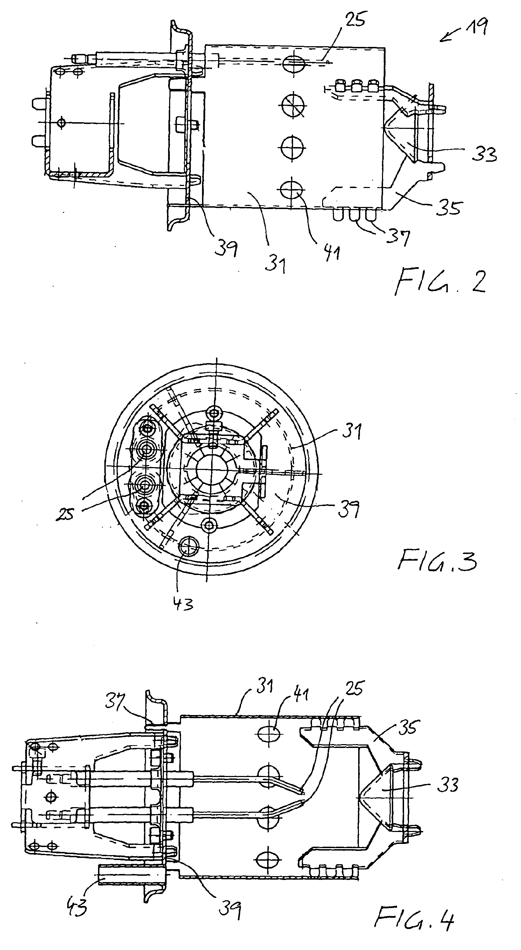

Der Verdampfer 19 ist in den Figuren 2 bis 4 dargestellt. Er weist ein Verdampferrohr 31 und einen Flammenteiler 33 auf. Der Flammenteiler 33 ist mit drei Beinen 35 am Verdampferrohr befestigt. Die Befestigung geschieht über Blechlappen 37, die an den Beinen 35 ausgeformt sind und in Schlitzen am Verdampferrohr 31 stecken. Diese eingesteckten Blechlappen 37 können um 20 bis 30° verdreht werden und sind dadurch mit dem Verdampferrohr 31 mechanisch fest verbunden. Ebenso ist das Verdampferrohr 31 an der Stauscheibe 39 mittels drei in Schlitzen steckenden und verdrehten Blechlappen 37 befestigt. Dadurch ist das Verdampferrohr 31 sehr einfach in die Stauscheibe 39 einsetzbar und wieder von der Stauscheibe 39 entfernbar.

Im Verdampferrohr sind Abgasrezirkulationsöffnungen 41 vorhanden. Der vom Verdampferrrohr umschlossene Raum dient der Durchmischung von Brennstoffdampf, Frischluft und rezirkulierten, sauerstoffarmen Verbrennungsgasen. Der durch die Düse 15 in diesen Raum eingespritze Brennstoff vergast in diesem Raum und brennt danach mit einer blauen Flamme mit relativ niedriger Temperatur und schadstoffarm.

Für die Zündung des Brennstoffes sind zwei Elektroden 25 vorhanden, die durch die Stauscheibe 39 hindurchgeführt sind. In der Stauscheibe 39 ist zudem eine Öffnung 43 für einen Temperaturfühler vorhanden.

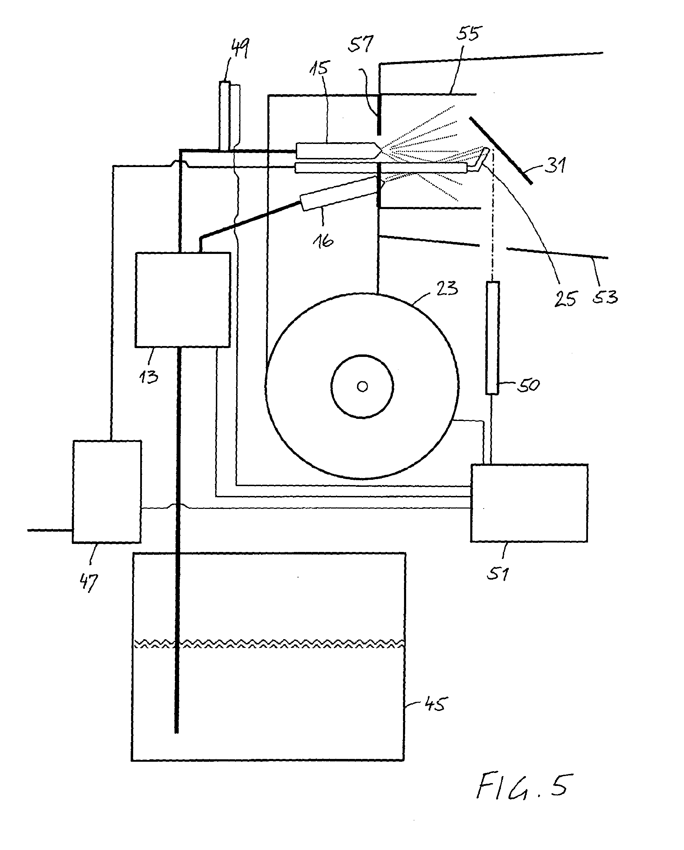

In der Figur 5 ist schematisch eine vorteilhafte Vorrichtung zur Prüfung von flüssigem Brennstoff, insbesondere Heizöl auf dessen potentielle erosive Wirkung beim Verbrennen in einer Heizungsanlage dargestellt. Diese weist einen Brennstoffbehälter 45 für eine Brennstoffprobe, eine an den Brennstoffbehälter 45 angeschlossene Brennstoffpumpe 13 und zwei nach der Brennstoffpumpe 13 angeordnete Düsen 15, 16 auf. Die erste Düse 15 weist eine Kegelcharakteristik mit einem üblichen Winkel zum Versprühen von Brennstoff auf. Die zweite Düse 16 spritzt Brennstoff gezielt auf die Prüffläche 31. Sie weist dazu eine Charakteristik mit einem spitzen Kegelwinkel von wenigen Graden auf. Ferner ist ein Spannungswandler 47 und eine mit dem Spannungswandler 47 verbundene Elektrode 25 vorhanden. Ein Ventilator 23 für eine Frischluftzufuhr und eine Steuerung 51 zur Steuerung der Brennstoffpumpe 13, des Spannungswandlers 47 und des Ventilators 23 komplettieren den Prüfapparat. Weiter ist in der Vorrichtung zum Prüfen von flüssigem Brennstoff eine ersetzbare, metallene Prüffläche 31 im Bereich der Flamme angeordnet. Diese ist geerdet. Mit einem Temperaturfühler 49 kann die Öltemperatur überwacht werden. Mit einem Infrarotfühler 50 kann die Temperatur der Prüffläche 31 überwacht werden.

Für die Flamme ist ein Brennraum innerhalb einer Brennraumwandung 53 vorhanden, dessen Ausgestaltung von untergeordneter Bedeutung ist. Damit die Flamme ruhig brennt, ist ein Flammrohr 55 vorhanden. Die Frischluft wird durch das Gebläse 23 gegen eine Stauscheibe 57 geblasen. Diese besitzt zentral eine Luftöffnung, in der die erste Düse derart angeordnet ist, dass rings um die erste Düse 15 eine Ringöffnung vorliegt. Axial in der Strömungsrichtung der Luft ist die Prüffläche 31 angeordnet. In der Nähe und vor der Prüffläche 31 ist eine Elektrode 25 angeordnet, die mit beispielsweise 12'000 Volt Gleich- oder Wechselspannung aus dem Stromwandler 47 versehen werden kann. Die zweite Düse 16 ist auf die Prüffläche 31 gerichtet, und zwar so, dass der durch die zweite Düse eingespritzte Brennstoff etwa zentral auf die Prüffläche 31 auftrifft und diese benetzt. Somit ist diese Stelle der Prüffläche 31 durch den Luftstrom und den benetzenden Brennstoff gekühlt. Sie liegt in unmittelbarer Nähe zum bzw. im dem Spannungsfeld stark ausgesetzten Bereich des Plasmas. Diese Stelle wird auch durch den Infrarotfühler überwacht.

Die Steuerung 51 kann nach einem Zeitgeber regelmässig den Ventilator 23, die Ölpumpe 13, eventuell ein nicht dargestelltes Ventil zur Regelung der Düsen 15,16 und den Stromwandler 47 ansteuern. Sie kann diese aber auch aufgrund von Messewerten ansteuern. Als Messwert kommt insbesondere die Temperatur der Prüffläche 31 in Frage. So kann die Prüfverfahrens-Sequenz gestartet werden, sobald der Temperaturfühler der Prüffläche beispielsweise 250, 300, 350 oder 380 Grad misst. Die Sequenz kann beendet werden, wenn die Prüffläche 550, 600 oder 650 Grad misst.

Wesentlich an der Prüfungsvorrichtung ist ihre konstant gleichbleibende Ausbildung, die eine Vergleichbarkeit der Resultate gewährleistet.

In einer Prüfungsvorrichtung kann auch der Brennstoff auf eine Prüffläche aufgebracht werden, die elektrisch vorgewärmt ist. Dazu kann die Prüffläche aus einem Alloy-Streifen bestehen, der an eine Stromquelle angeschlossen ist und so direkt geheizt ist oder mit einer Heizwicklung indirekt geheizt ist. Der Brennstoff kann auf die Prüffläche aufgegossen, aufgetropft oder aufgesprüht werden. Die Verdunstung oder Verdampfung des Brennstoffes erfolgt dank der Wärmeenergiezufuhr zur Prüffläche. Dies erlaubt, die Prüffläche kontrolliert einen gewünschten Temperaturverlauf wiederholt durchlaufen zu lassen.

### Beispiele eines Ablaufes eines Prüfverfahrens:

Mit einem in den Figuren dargestellten Brenner 11 wird eine Brennstoffprobe geprüft. Es sind jedoch auch andere Prüfungsvorrichtungen möglich und sinnvoll. Zur Prüfung des Brennstoffes wird der Brennstoff mit der Düse 15 in das Vergaserrohr 31 gesprüht. Im Vergaserrohr wird der Brennstoff dank vorhandener Restwärme oder der mit den Elektroden zugeführten Energie vergast und am Zündfunken entflammt. Mit den Zündelektroden wird das Plasma einem elektrischen Spannungsfeld von zweimal 7500 Volt Wechselspannung ausgesetzt. Dank dem niedrigen Schwefelgehalt, der Gegenwart der mit dem Gebläse eingeblasenen sauerstoffreichen Frischluft und dem elektrischen Spannungsfeld sind die Verbrennungsbedingungen derart, dass am Vergaserrohr 31 sehr rasch erodierte Stellen entstehen.

In der Versuchsphase werden Zyklen von 15 Minuten gewählt, um eine Reproduzierbarkeit der Versuchsreihe zu gewährleisten. Der Versuch wird mit dem Brenner gemäss Figuren 1 bis 4 ausgeführt. Es wird während etwa 15 Sekunden vorgezündet. Danach wird bei laufender Frischluftzufuhr das zu prüfende Heizöl eingespritzt. Es benetzt die innere Oberfläche des Vergaserrohrs. Sofort entzündet es sich am Zündfunken. Das entstehende Plasma wird während der gesamten Brenndauer dem elektrischen Spannungsfeld zwischen den Elektroden ausgesetzt. Während 5 Minuten wird die Flamme so aufrechterhalten und einer Potenzialdifferenz zwischen der Prüffläche und der Elektrode ausgesetzt. Danach wird die Ölzufuhr abgestellt und nachgelüftet. Die Nachlüftzeit beträgt 10 Minuten. Dann beginnt der Zyklus von vorne.

Mit diesem Zyklus sind 20 Minuten Brenndauer innerhalb einer Stunde der Versuchszeit erosionswirksam. Nach spätestens 120 Stunden oder 5 Tagen Versuchszeit liegt ein zuverlässiges Prüfungsergebnis vor. Das Verdampferrohr 31 wird ausgebaut und überprüft. Das Ausmass der Erosion gibt Aufschluss über die erosive Wirkung des Heizöls der geprüften Charge in Heizungsanlagen. Das Verdampferrohr wird zum Beleg der Brennstoffqualität beschriftet und archiviert.

Es ist zu erwarten, dass mit kürzeren Zyklen von 3 Minuten Brenndauer und 6 Minuten Nachlüftzeit die Versuchszeit verkürzt werden kann. Dies ist darauf zurückzuführen, dass die Erosion in der kühleren Startphase stärker auftritt, solange der Brennstoff die Prüffläche noch flüssig erreicht. Durch geeignete Massnahmen zur Verkürzung der Nachlüftzeit kann die Versuchszeit zusätzlich verkürzt werden.

Eine bevorzugte Sequenz weist 2 Minuten Brenndauer auf, die von 3 Minuten Nachlüften gefolgt ist. Die Vorzündzeit übergreift die Nachlüftzeit und ist kurz, beispielsweise 2 Sekunden. Danach setzt die Brennstoffpumpe für 2 Minuten ein, während denen die geerdete Prüffläche besprüht wird. Während der gesamten Zeit der Ölzufuhr wird eine Spannung von ca. 15'000 Volt gegenüber der Erde auf die einzige Elektrode gegeben. Die Erosivität des Brennstoffes kann bei dieser Prüfungsanlage nach ca. 3 Tagen der Versuchsdauer beurteilt werden.

## Patentansprüche

1. Verfahren zur Vermeidung von Erosionsschäden in Heizungsanlagen in Folge einer Verbrennung von schwefelarmem Brennstoff, insbesondere Dieselöl oder Heizöl, mit einem Massenanteil an Schwefel von höchstens 0,05% in einem Verbrennungsraum der Heizungsanlage, **dadurch gekennzeichnet, dass** auf den Schaden verursachenden Prozess Einfluss genommen wird, indem eine während der Verbrennung des Brennstoffs im Verbrennungsraum vorhandene elektrische Spannung gering gehalten oder vermindert wird.

2. Verfahren zur Vermeidung von Erosionsschäden in Heizungsanlagen in Folge einer Verbrennung von schwefelarmem Brennstoff, insbesondere Dieselöl oder Heizöl, mit einem Massenanteil an Schwefel von höchstens 0,05% in einem Verbrennungsraum der Heizungsanlage, insbesondere nach Anspruch 1, **dadurch gekennzeichnet, dass** dem schwefelarmen Brennstoff ein Additiv zugesetzt wird.

3. Verfahren nach Anspruch 1 oder 2, **dadurch gekennzeichnet, dass** dem Brennstoff ein Additiv zum Verhindern oder Abbauen von elektrostatischen Aufladungen (Static Dissipator Additive) zugesetzt wird.

4. Verfahren nach einem der vorangehenden Ansprüche, **dadurch gekennzeichnet, dass** auf den Schaden verursachenden Prozess Einfluss genommen wird, indem die Bildung von Carbonsäure während der Verbrennung des Brennstoffs im Verbrennungsraum vermindert wird.

5. Verfahren nach Anspruch 4, **dadurch gekennzeichnet, dass** dem schwefelarmen Brennstoff ein Antioxidans zugesetzt wird.

6. Flüssiger schwefelarmer Brennstoff, insbesondere Dieselöl oder Heizöl, mit einem Massenanteil an Schwefel von höchstens 0,05%, **gekennzeichnet durch** ein dem Brennstoff zugesetztes Additiv zum Verhindern oder Abbauen von elektrostatischen Aufladungen (Static Dissipator Additive).

7. Flüssiger schwefelarmer Brennstoff, insbesondere Dieselöl oder Heizöl, mit einem Massenanteil an Schwefel von höchstens 0,05%, insbesondere nach Anspruch 6, **gekennzeichnet durch** ein dem Brennstoff zugesetztes Antioxidans.

8. Verwendung eines Additivs in einem Brennstoff, insbesondere Dieselöl oder Heizöl, mit einem Massenanteil an Schwefel von höchstens 0,05% zur Vermeidung von Erosionsschäden in Heizungsanlagen in Folge einer Verbrennung des schwefelarmen Brennstoffs in einem Verbrennungsraum der Heizungsanlage.

9. Verfahren zur Vermeidung von Erosionsschäden in Heizungsanlagen in Folge einer Verbrennung von Brennstoff, insbesondere Dieselöl oder Heizöl, mit einem Massenanteil an Schwefel von höchstens 0,05% in einem Verbrennungsraum der Heizungsanlage, **dadurch gekennzeichnet, dass** auf den Schaden verursachenden Prozess Einfluss genommen wird, indem die Bildung von Carbonsäure während der Verbrennung des Brennstoffs im Verbrennungsraum vermindert wird.

10. Verfahren nach Anspruch 9, **dadurch gekennzeichnet, dass** dem schwefelarmen Brennstoff ein Antioxidans zugesetzt wird.
